# EUROPEAN PATENT SPECIFICATION

(11) **EP 3 119 474 B1**
(45) Date of publication and mention of the grant of the patent: **23.09.2020**
(21) Application number: 15709694.2
(22) Date of filing: 16.03.2015
(51) Int. Cl.: A61N 7/02, A61B 18/18, A61B 18/00, A61B 90/00, A61B 34/10

(54) **GUIDED THERMAL TREATMENT SYSTEM**
GEFÜHRTES WÄRMEBEHANDLUNGSSYSTEM
SYSTÈME DE TRAITEMENT THERMIQUE GUIDÉ

(30) Priority: 21.03.2014 EP 14161041
(43) Date of publication of application: 25.01.2017
(73) Proprietor: Koninklijke Philips N.V., 5656 AG Eindhoven (NL)
(72) Inventor: TANTTU, Jukka Ilmari, NL-5656 AE Eindhoven (NL); YLIHAUTALA, Mika Petri, NL-5656 AE Eindhoven (NL); KOSKELA, Ilpo Asko Julius, NL-5656 AE Eindhoven (NL)
(74) Representative: Cohen, Julius Simon
(86) International application number: PCT/EP2015/055381
(87) International publication number: WO 2015/140083

(56) References cited:
- EP-A2- 2 662 041
- WO-A1-2007/047247
- WO-A1-2010/029474
- WO-A1-2010/029479
- US-A1- 2003 181 965
- US-A1- 2010 179 538

## Description

### FIELD OF THE INVENTION

The invention relates to a system in the field of guided thermal treatment and more specifically to the temperature control.

### BACKGROUND OF THE INVENTION

In high-intensity focused ultrasound (HIFU) therapy ultrasound (US) focus is positioned within the target lesion to be heated. The aim is typically to ablate a lesion, but there are applications where non-ablative heating is enough.

Typically a target is heated by applying thermal treatment pulse to it, whereby the target is exposed for a relative short time (order 10-100 s) to a high intesity utrasound wave. This energy application is called sonication. Besides the heating of the target the ultrasound beam also heats tissues outside the target. Although the heating of a single sonication in the non-targeted area is typically too low to produce tissue damage, the heat accumulation of several sonications may cause safety issues. Typical adverse event of HIFU is skin burn in the area between US transducer and the target (s.c. near field (NF) volume). Therefore, cool-down periods are needed between sonications to let the normal tissues to cool via normal body cooling processes (e.g. diffusion, perfusion, radiation). This cool-down period reduces the treatment efficiency.

Document WO2010029474A1 describes an MR guided thermal treatment system in which the cool-down period is regulated in dependence of the off-focus maximum temperature during the energy deposit preceding the cool-down period. The maximum temperature rise in the off-focus region is approximately linearly dependent on the deposited energy density and a measurement of the maximum temperature can therefore be used to set the cool-down period. The linear dependence appears to be valid when the temperature decrease due to diffusion of heat can be neglected in the middle of the off-focus ultrasound cone during heating.

WO2010/029479 describes a therapy system to perform successive deposits of energy in a target zone. The therapy system is configured to produce an a priori estimate of a the cool-down period on the basis of estimated induced heating.

### SUMMARY OF THE INVENTION

It is an insight of the invention that to achieve good treatment efficiency there is a need to minimize the cooling times and while preventing overheating of a subject treated.

This object is achieved by a guided thermal treatment system as described in claim 1.

This object is also achieved by a computer program product as claimed in claim 5.

HIFU systems software (SW) have typically algorithms to estimate the near field (NF) heat accumulation and corresponding cooling time needed to keep the safety risks low. NF algorithms can be based on theoretical calculation models and can utilize information from the MRI temperature maps. MRI temperature map information is, however, typically incomplete. The proton resonance frequency shift (PRFS) method measures relative temperature changes and typically does not have cumulative heating information. Furthermore, PRFS method works only in water containing tissues, and thus it typically gives information from the muscle, while the subcutaneous fat and skin (due to thin thickness) are not monitored. Alternative T2 based methods can give information on the fat temperature, but this method does not work in other tissue types. Consequently, sufficient on-line monitoring of NF heating is challenging, and typically calculation models are needed for safety algorithms.

Calculation models estimating heat accumulation in NF area need to take into account the timing and energy of the sonications as well as on the positioning of the ultrasound beam path. In an optimal treatment the next sonication is started as soon as possible. This requires predictive algorithms estimating the heating effect of the coming sonication. This can be difficult task, especially, when feedback sonications, i.e. sonications where the duration and/or power of the sonication are adjusted based on the temperature feedback, are used. Besides these technical factors, the heat accumulation estimation needs to model the heating and cooling of the patient near field. There are individual patient dependent variations; patients have e.g. different capacity handle heat, and they have different thickness of subcutaneous fat layer affecting the heat diffusion. Therefore the heat accumulation algorithm are always approximation, and the physician should be allowed to judge the necessary cooling time based on the medical information available, based on the communication with the patient, and based on his/her estimate for the risk/benefit ratio.

SW heat accumulation algorithm might be conservative and prevent sonication even in the case of very small probability of adverse event. This would easily lead to suboptimal patient treatment for most patients. If the algorithm is less conservative, the user does not get any support from the system for patients with higher risk.

In order to solve this issue, the invention proposes use of one or more two-level cool-down periods.

Software estimates energy accumulation in the near field volume based on the sonication history and the user selection for the next sonication (e.g. by known thermal models). User selections may be e.g. US power, cell size, US frequency, cell location (affecting ultrasound beam path location). The algorithm provides two level of cool-down periods by splitting a (total) cool-down period in a
- mandatory part of the cool-down period to prevent sonications with significantly increased risk of adverse events. A mandatory part of the cool-down period cannot be overwritten by the user. In principle, the duration of the mandatory part of the cool-down period should be enough to prevent overheating in a non-sensitive patient (e.g. having normal or increased thermoregulatory capability).
- recommended part of the cool-down period indicating that there is an elevated risk for sensitive patients (e.g. having reduced thermoregulatory capability) when sonicating during this recommended part. This part of the total cool-down period can be overwritten by the user based on his/her judgment about medical information available and risk/benefit analysis. Overwriting could for example be done by simply pressing a "start sonication" button. Also, overwriting could be done by a manual adjustment of a remaining duration of the recommended part of the cool-down period. Together the mandatory part and recommended part of the cool-down period are the (total) cool-down period.

The user could be informed about the durations of the mandatory and recommended part of the cool-down period by means of an audio/visual display. This information can be visualized to the user e.g. by using cooling bar with color code indication mandatory and recommended part of the cooling time.

By using a mandatory part of a cool-down period in combination with a recommended part of a cool-down period a safe treatment can be obtained, while decreasing a total cool-down period and thereby reducing unnecessary treatment delays.

The estimated cooling time before the next sonication depends on the parameters of the next sonication. The user may vary these parameters in planning phase and define e.g. the order of the sonications based on the estimated cooling time.

This invention can be used e.g. in HIFU systems, or other therapy devices providing non-invasively local heating (e.g. RF ablation).

### BRIEF DESCRIPTION OF THE FIGURES

Figure 1 illustrates diagrammatically a magnetic resonance guided thermal treatment system in which the invention is used.
Figure 2 schematically shows one possible implementation of the invention.

### DETAILED DESCRIPTION OF THE INVENTION

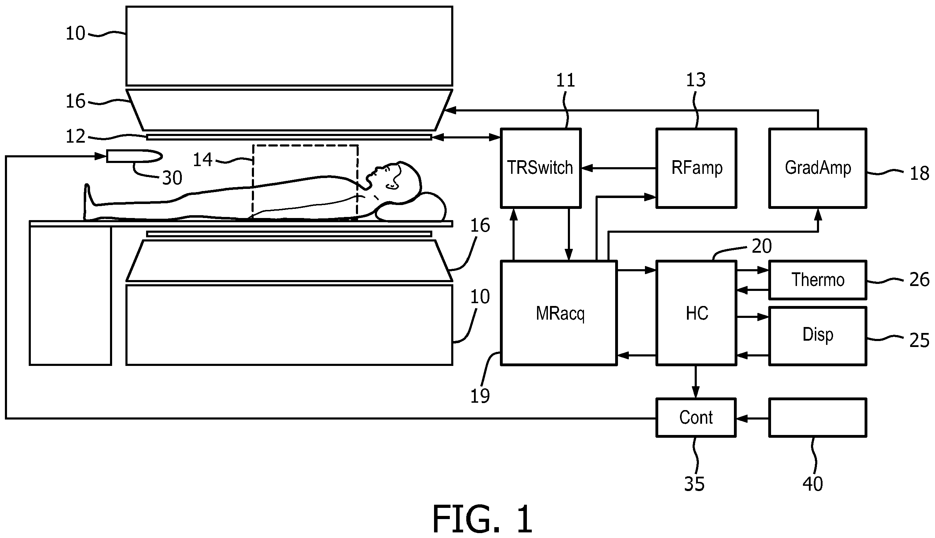

Figure 1 illustrates diagrammatically an embodiment of a guided thermal treatment system in which the invention is used. The embodiment shown in figure 1 is an MR guided intervention system comprises a magnetic resonance system (not entirely shown) and a thermal therapy system 30. The invention is not limited to systems using MR for the guidance. Other technologies for guidance, wherein information about the temperature of the area exposed by the thermal pulses can be derived can be used. The thermal therapy system 30 could be anything used for MR guided thermal treatment. Thermal treatment could for example be applied by means of HIFU or by means of a microwave antenna. The thermal treatment system is configured for applying thermal treatment pulses, which are spaced by a cool-down period. The thermal treatment system can be turned on or off by a controller 35.

The magnetic resonance examination system comprises a main magnet 10 which generates a steady homogeneous main magnetic field within the examination zone 14. This main magnetic field causes a partial orientation of the spins in the object to be examined along the field lines of the main magnetic field. An RF system is provided with one or more RF antennae 12 to emit an RF excitation electromagnetic field into the examination zone 14 to excite spins in the body of the object to be examined. The relaxing spins emit magnetic resonance signals in the RF range which are picked up by the RF antennae 12, notably in the form of RF receiving coils. The RF system 12 is coupled to an Tx/Rx switch 11, which in turn is coupled to an RF amplifier 13. Further, gradient coils 16 are provided to generate temporary magnetic gradient fields, notably read gradient pulses and phase encoding gradients. These gradient fields usually are orientated in mutual orthogonal directions and impose spatial encoding on the magnetic resonance signals. Gradient amplifiers 18 are provided to activate the gradient coils 16 to generate the magnetic gradient encoding fields. The magnetic resonance signals picked up by the RF receiver antennae 12 are applied to an MRI data acquisition system which includes a spectrometer 19. The MR protocol used, determines a contrast type (e.g. T1 weighted or T2 weighted) of the acquired data. The MRI data acquisition system 19 provides the data to a host computer 20. From the magnetic resonance signals an image can be reconstructed. The image can be displayed on a display 25. The display can also be used to display information on the mandatory and recommended part of the cool-down period.

The magnetic resonance examination system comprises a thermal module 40. The thermal module uses one or more thermal models to estimate heat accumulation in the near-field area, which take into account the timing and energy of sonications as well as the positioning of the ultrasound beam path. Such models are well known in the art and can be used to determine a duration of the cool-down period. By using non-conservative (e.g. values representative for an average patient) model parameter values e.g. for tissue's heat capacity, perfusion, thickness of fat layer a duration of the mandatory part of the cool-down period can be calculated. By using more conservative values for the model parameters a duration of the recommended part of the cool-down period can be calculated. The choice for the model parameter used to calculate the mandatory and recommended part of the cool-down period depends on a preference on where to put a trade-off between safety and flexibility for the user.

Alternatively, the duration of the recommended part of the cool-down period can be a percentage of the duration of the mandatory part of the cool-down period.

Also the thermal module may be a separate software module apart from the magnetic resonance system. Such thermal module may be used to upgrade a magnetic resonance examination system or may be used to calculate a duration of a mandatory and recommended part of the cool-down period off-line, for example as part of a thermal treatment planning procedure.

The results of the thermal model can be combined with one or more temperature measurements. These temperature measurements can be used to adjust the mandatory and/or recommended part of the cool-down period.

In one embodiment, the invention comprises a thermometry module 26, which derives a temperature distribution from the MR signals. The temperature distribution may be a qualitative distribution. The temperature distribution may for example be a result of a comparison between T1, T2 or T2* based signals acquired prior to thermal treatment and signals of the same contrast type acquired during the cool-down period.

In one embodiment the temperature distribution is displayed to a user by means of a display 25. Based on the displayed temperature distribution, the user can decide whether or not to start with a new sonication pulse during the recommended part of the cool-down period.

According to one embodiment, the magnetic guided thermal treatment system is configured to check if the temperature dependent magnetic resonance signals acquired during the cool-down period are similar to temperature dependent magnetic resonance signals of the same contrast type acquired prior to the thermal treatment. In case these signals are similar, the temperature will also be similar to the temperature at the start of treatment

According to one embodiment, the temperature dependent signals are acquired using relaxation time constant based thermometry (e.g. T1, T2, T2*) in order to determine the temperature or temperature change in a fat layer. According to another embodiment, the temperature dependent signals are acquired using spectroscopic techniques or multi-echo techniques. These techniques are sensitive to temperature changes on the border between muscle tissue and fat. Those techniques could potentially also be applied to monitor cooling during brain ablation through NAA-PRF spectroscopic thermometry. With the spectroscopic or multi-echo PRF techniques multiple spectral peaks can be resolved and knowing the temperature dependency of the peaks, the frequency difference between the peaks can be converted to an absolute temperature estimate.

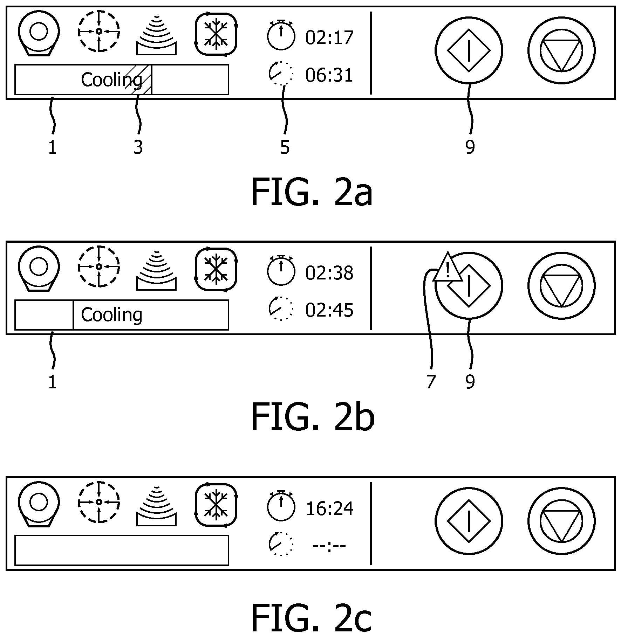

Figure 2 schematically shows one possible implementation of an user interface for use in an embodiment of the the invention. The thermal module is configured to divide the total cool-down period in a first period, indicatedin figure 2 by a bar 1 and a second period, indicated in figure 2 by a bar 3. These bars may be displayed on display 25, figure 1. These bars could for example be distinguishable based on their colour. The bars get shorter with time, length indicating remaining cool-down period. Bar 3 represents mandatory part of the cool-down period during which the sonication is prevented (start sonication button is disabled (9 in figure 2a). Bar 1 indicates recommended cool-down. In figure 2a there is total cooling time of 6:31 5, of which about one minute is mandatory. After few minutes the mandatory part is elapsed and the remaining bar 1 indicates the recommended part of the cool-down period. This could for example be recognized by the colour of the bar. Now user may start sonication if based on his patient specific judgement the treatment risk is low. The extra care required in sonication started before the recommended part of the cool-down period could be shown 7 at the start sonication button 9 e.g. by a yellow square or other symbol, color see Figure 2b. After few more minutes all the cooling bar has disappeared indicating that the recommended cooling time is over and the risks in near field volume are low (figure 2c).

The estimated cooling times are sonication dependent. Each time the user plans a new sonication, select one of already planned, or changes the sonication parameters cool-down period appearance is updated, and the user get immediate response for the cool-down periods. According to embodiments of the invention, the cool-down periods are estimated based on
- Sonication history data such as, power, duration, frequency, location and timing of all executed sonications
- Sonication prediction data such as, power, expected duration, frequency and location of the next selected sonication
- Patient model including temperature cooling and ultrasound absorption models for skin, fat and abdominal muscle
- Environment model including temperature or temperature estimate of the patient contact
- Ultrasound model including spatial ultrasound intensity data

Whilst the invention has been illustrated and described in detail in the drawings and foregoing description, such illustrations and description are to be considered illustrative or exemplary and not restrictive; the invention is defined in the appended claims and can be used for temperature control within the field of MR guided thermal treatment.

## Claims

1. A guided thermal treatment system, comprising:
- a thermal treatment system (30) configured for applying thermal treatment pulses to a patient, wherein the thermal treatment pulses are spaced in time by a cool-down period,
- a guidance system, configured to provide guidance to the thermal treatment, wherein the guidance system comprises a thermal module (40) configured for calculation of the cool-down period, **characterized in that** the thermal module is configured to calculate the cool-down period by
- calculation of a duration of a mandatory part of the cool-down period based on a thermal model using non-conservative patient model parameter values, wherein the model takes into account timing and energy of sonications as well as a positioning of an ultrasound beam path, wherein the mandatory cool-down period indicates a period during which use of a thermal treatment system is prevented;
- calculation of a duration of a recommended part of the cool-down period, wherein the recommended part of the cool-down period indicates a period during which thermal pulse would result in an elevated risk of overheating in a sensitive patient, but not in a non-sensitive patient;

2. A guided thermal treatment system as claimed in claim 1 , wherein the guidance system is a magnetic resonance examination system.

3. A guided thermal treatment system as claimed in one of claims 1-2 comprising an audio and/or visual display configured to provide the user with information regarding the duration of the mandatory and recommended part of the cool-down period.

4. A guided thermal treatment system as claimed in any of claims 1-3 configured to update a duration of the mandatory and/or recommended part of the cool-down period based on a temperature measurement performed by the magnetic resonance system.

5. Computer program product comprising program code means for calculation of a cool-down period, wherein a cool-down period is a period in between thermal treatment pulses in order to prevent overheating of a patient undergoing the thermal treatment **characterized in that** the computer program product is configured to when executed on a processor of the treatment system according to claim 1, carry out the steps
- calculation of a duration of a mandatory part of the cool-down period based on a thermal model using non-conservative patient model parameter values, wherein the model takes into account timing and energy of sonications as well as a positioning of an ultrasound beam path, wherein the mandatory cool-down period indicates a period during which use of a thermal treatment system is prevented;
- calculation of a duration of a recommended part of the cool-down period,
wherein the recommended part of the cool-down period indicates a period during which thermal pulse would result in an elevated risk of overheating in a sensitive patient, but not in a non-sensitive patient;

6. Computer program product comprising program as claimed in claim 5 configured means for causing a computer to control an apparatus as claimed in claim 1 by
- preventing a magnetic resonance guided thermal treatment system from applying thermal treatment pulses during the mandatory part of the cool-down period and
- allowing the user to overwrite the duration of the recommended part of the cool-down period.

## Patentansprüche

1. Geführtes Wärmebehandlungssystem, umfassend:
- ein Wärmebehandlungssystem (30), das zum Anlegen von Wärmebehandlungsimpulsen an einen Patienten konfiguriert ist, wobei die Wärmebehandlungsimpulse zeitlich durch eine Abkühlperiode beabstandet sind;
- ein Leitsystem, das konfiguriert ist, um eine Führung für die Wärmebehandlung bereitzustellen, wobei das Leitsystem ein Wärmemodul (40) umfasst, das zur Berechnung der Abkühlperiode konfiguriert ist, **dadurch gekennzeichnet, dass** das Wärmemodul zur Berechnung der Abkühlperiode konfiguriert ist, durch
- Berechnung einer Dauer eines obligatorischen Teils der Abkühlperiode auf der Grundlage eines Wärmemodells unter Verwendung nicht konservativer Parameterwerte des Patientenmodells, wobei das Modell den Zeitpunkt und die Energie der Beschallung sowie die Positionierung eines Ultraschallstrahlengangs berücksichtigt, wobei die obligatorische Abkühlperiode eine Periode angibt, während der die Verwendung eines Wärmebehandlungssystems verhindert wird;
- Berechnung einer Dauer eines empfohlenen Teils der Abkühlperiode, wobei der empfohlene Teil der Abkühlperiode eine Periode angibt, während der ein thermischer Impuls bei einem empfindlichen Patienten zu einem erhöhten Überhitzungsrisiko führen würde, nicht jedoch bei a unempfindlicher Patient.

2. Geführtes Wärmebehandlungssystem nach Anspruch 1, wobei das Leitsystem ein Magnetresonanzuntersuchungssystem ist.

3. Geführtes Wärmebehandlungssystem nach einem der Ansprüche 1 bis 2, umfassend eine Audio- und/oder visuelle Anzeige, die konfiguriert ist, um dem Benutzer Informationen über die Dauer des obligatorischen und empfohlenen Teils der Abkühlperiode bereitzustellen.

4. Geführtes Wärmebehandlungssystem nach einem der Ansprüche 1 bis 3, das konfiguriert ist, um eine Dauer des obligatorischen und/oder empfohlenen Teils der Abkühlperiode basierend auf einer vom Magnetresonanzsystem durchgeführten Temperaturmessung zu aktualisieren.

5. Computerprogrammprodukt, umfassend Programmcodeeinrichtung zur Berechnung einer Abkühlperiode, wobei eine Abkühlperiode eine Periode zwischen Wärmebehandlungsimpulsen ist, um eine Überhitzung eines Patienten zu verhindern, der sich der Wärmebehandlung unterzieht, **dadurch gekennzeichnet, dass** das Computerprogrammprodukt konfiguriert ist, um, wenn es auf einem Prozessor des Behandlungssystems gemäß Anspruch 1 ausgeführt wird, die Schritte auszuführen von:
- Berechnung einer Dauer eines obligatorischen Teils der Abkühlperiode auf der Grundlage eines Wärmemodells unter Verwendung nicht konservativer Parameterwerte des Patientenmodells, wobei das Modell den Zeitpunkt und die Energie der Beschallung sowie die Positionierung eines Ultraschallstrahlengangs berücksichtigt, wobei die obligatorische Abkühlperiode eine Periode angibt, während der die Verwendung eines Wärmebehandlungssystems verhindert wird;
- Berechnung einer Dauer eines empfohlenen Teils der Abkühlperiode, wobei der empfohlene Teil der Abkühlperiode eine Periode angibt, während der ein thermischer Impuls bei einem empfindlichen Patienten zu einem erhöhten Überhitzungsrisiko führen würde, nicht jedoch bei a unempfindlicher Patient.

6. Computerprogrammprodukt, umfassend ein Programm nach Anspruch 5, konfigurierte Mittel, um einen Computer zu veranlassen, eine Vorrichtung nach Anspruch 1 zu steuern, durch:
- Verhindern, dass ein magnetresonanzgeführtes Wärmebehandlungssystem während des obligatorischen Teils der Abkühlperiode Wärmebehandlungsimpulse anlegt und
- Ermöglichen, dass der Benutzer die Dauer des empfohlenen Teils der Abkühlperiode überschreibt.

## Revendications

1. Système de traitement thermique guidé, comprenant:
- un système de traitement thermique (30) configuré pour appliquer des impulsions de traitement thermique à un patient, où les impulsions de traitement thermique sont espacées dans le temps par une période de refroidissement,
- un système de guidage, configuré pour fournir un guidage vers le traitement thermique, où le système de guidage comprend un module thermique (40) configuré pour le calcul de la période de refroidissement, **caractérisé en ce que** le module thermique est configuré pour calculer la période de refroidissement par
- calcul d'une durée d'une partie obligatoire de la période de refroidissement sur la base d'un modèle thermique utilisant des valeurs de paramètres de modèle de patient non conservatrices, où le modèle prend en compte le temps et l'énergie des sonications ainsi que le positionnement d'un trajet de faisceau ultrasonore, où le période de refroidissement obligatoire indique une période pendant laquelle l'utilisation d'un système de traitement thermique est empêchée;
- calcul d'une durée d'une partie recommandée de la période de refroidissement, où la partie recommandée de la période de refroidissement indique une période pendant laquelle l'impulsion thermique résulterait en un risque élevé de surchauffe chez un patient sensible, mais pas chez un patient non sensible.

2. Système de traitement thermique guidé selon la revendication 1, dans lequel le système de guidage est un système d'examen par résonance magnétique.

3. Système de traitement thermique guidé selon l'une des revendications 1 à 2, comprenant un affichage audio et/ou visuel configuré pour fournir à l'utilisateur des informations concernant la durée de la partie obligatoire et recommandée de la période de refroidissement.

4. Système de traitement thermique guidé selon l'une quelconque des revendications 1 à 3, configuré pour mettre à jour une durée de la partie obligatoire et/ou recommandée de la période de refroidissement sur la base d'une mesure de la température effectuée par le système de résonance magnétique.

5. Produit de programme informatique comprenant des moyens de code de programme pour le calcul d'une période de refroidissement, où une période de refroidissement est une période entre les impulsions de traitement thermique afin d'éviter la surchauffe d'un patient subissant le traitement thermique **caractérisé en ce que** le produit de programme informatique est configuré pour, lorsqu'il est exécuté sur un processeur du système de traitement selon la revendication 1, effectuer les étapes de
- calcul d'une durée d'une partie obligatoire de la période de refroidissement sur la base d'un modèle thermique utilisant des valeurs de paramètres de modèle de patient non conservatrices, où le modèle prend en compte le temps et l'énergie des sonications ainsi que le positionnement d'un trajet de faisceau ultrasonore, où la période de refroidissement obligatoire indique une période pendant laquelle l'utilisation d'un système de traitement thermique est empêchée;
- calcul d'une durée d'une partie recommandée de la période de refroidissement, où la partie recommandée de la période de refroidissement indique une période pendant laquelle l'impulsion thermique résulterait en un risque élevé de surchauffe chez un patient sensible, mais pas chez un patient non sensible.

6. Produit de programme informatique comprenant un programme selon la revendication 5, des moyens configurés pour amener un ordinateur à commander un appareil selon la revendication 1 par
- empêchement à un système de traitement thermique guidé par résonance magnétique d'appliquer des impulsions de traitement thermique pendant la partie obligatoire de la période de refroidissement et
- permission à l'utilisateur d'écraser la durée de la partie recommandée de la période de refroidissement.
